(19) Europäisches Patentamt
European Patent Office
Office européen des brevets

(11) **EP 3 174 571 B1**

(12) **EUROPÄISCHE PATENTSCHRIFT**

(45) Veröffentlichungstag und Bekanntmachung des Hinweises auf die Patenterteilung:
**28.08.2019 Patentblatt 2019/35**

(21) Anmeldenummer: **15739286.1**

(22) Anmeldetag: **20.07.2015**

(51) Int Cl.:
*A61M 1/16* (2006.01)    *A61M 1/36* (2006.01)

(86) Internationale Anmeldenummer:
**PCT/EP2015/066577**

(87) Internationale Veröffentlichungsnummer:
**WO 2016/016039 (04.02.2016 Gazette 2016/05)**

(54) **VORRICHTUNG ZUR ERKENNUNG DER RICHTUNG DER FLÜSSIGKEITSSTRÖMUNG DURCH EINEN DIALYSATOR**

DEVICE FOR IDENTIFYING THE DIRECTION OF LIQUID FLOW THROUGH A DIALYSER

DISPOSITIF DE DÉTECTION DU SENS DE L'ÉCOULEMENT DU LIQUIDE À TRAVERS UN DIALYSEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.08.2014 DE 102014011250**

(43) Veröffentlichungstag der Anmeldung:
**07.06.2017 Patentblatt 2017/23**

(73) Patentinhaber: **Fresenius Medical Care Deutschland GmbH**
**61352 Bad Homburg (DE)**

(72) Erfinder: **MAIERHOFER, Andreas**
**97442 Schweinfurt (DE)**

(74) Vertreter: **Oppermann, Frank**
**OANDO Oppermann & Oppermann LLP**
**Wilhelminenstrasse 1a**
**65193 Wiesbaden (DE)**

(56) Entgegenhaltungen:
**WO-A1-2012/159734    US-A1- 2013 338 560**

**Beschreibung**

Die Erfindung betrifft eine Vorrichtung zur Erkennung der Richtung der Flüssigkeitsströmung durch einen Dialysator, der eine von Blut durchströmte Blutkammer und eine von Dialysierflüssigkeit durchströmte Dialysierflüssigkeitskammer aufweist, die von einer semipermeablen Membran voneinander getrennt sind. Darüber hinaus betrifft die Erfindung eine extrakorporale Blutbehandlungsvorrichtung mit einem extrakorporalen Blutkreislauf, der die Blutkammer eines durch eine semipermeable Membran in die Blutkammer und eine Dialysierflüssigkeitskammer unterteilten Dialysators einschließt, und mit einem Flüssigkeitssystem, das die Dialysierflüssigkeitskammer des Dialysators einschließt, wobei die extrakorporale Blutbehandlungsvorrichtung eine Vorrichtung zur Erkennung der Richtung der Flüssigkeitsströmung durch den Dialysator aufweist.

Es sind verschiedene Arten von Blutbehandlungsvorrichtungen bekannt. Zu den bekannten Blutbehandlungsvorrichtungen zählen beispielsweise die Vorrichtungen zur Hämodialyse, Hämofiltration und Hämodiafiltration. Während der Blutbehandlung strömt das Blut des Patienten in einem extrakorporalen Blutkreislauf durch eine Blutbehandlungseinheit. Bei den Vorrichtungen zur Hämodialyse, Hämofiltration und Hämodiafiltration ist die Blutbehandlungseinheit ein Dialysator oder Filter, der durch eine semipermeable Membran in eine Blutkammer und eine Dialysierflüssigkeitskammer getrennt ist. Während der Blutbehandlung strömt das Blut durch die Blutkammer, während die Dialysierflüssigkeit durch die Dialysierflüssigkeitskammer strömt. Eine effektive Blutbehandlung setzt voraus, dass Blut und Dialysierflüssigkeit entlang der Membran des Dialysators oder Filters in entgegengesetzten Richtungen strömen. Bei gleicher Strömungsrichtung ist die Blutbehandlung weniger effektiv. Daher wird in der Praxis der Dialysator oder Filter nicht im Gleichstrom, sondern Gegenstrom betrieben. Allerdings kann in einzelnen Fällen auch eine reduzierte Dialyseeffizienz erwünscht sein, beispielsweise um ein Disequilibrium-Syndrom zu vermeiden. Dann kann ein Betrieb im Gleichstrom erfolgen, wenn die Dialyseeffizienz nicht durch eine Veränderung des Blutflusses oder Dialysierflüssigkeitsflusses verringert wird. Auch bei Problemen mit dem Dialysator (Clotting) kann ein Anschluss im Gleichstrom von Vorteil sein. Ferner besteht bei Behandlungen mit einer Kombination aus diffusivem und konvektivem Stoffaustausch (Hämodiafiltration) durch einen Betrieb im Gleichstrom die Möglichkeit der Beschränkung des diffusiven Anteils am Stoffaustausch.

Es sind verschiedene physikalische und/oder chemische Größen bekannt, mit denen die Leistungsfähigkeit eines Dialysators und/oder die Effektivität einer Dialysebehandlung angegeben werden können. Eine bekannte Größe zur Angabe der Effektivität einer Dialysebehandlung stellt die Clearance K dar. Die Clearance K einer Substanz ist der Teilstrom des Gesamtstroms durch den Dialysator, der von der betreffenden Substanz vollständig befreit wird. Für die Effektivität einer Dialysebehandlung ist die sogenannte Dialysedosis KT/V von entscheidender Bedeutung, die als der Quotient aus dem Produkt von Clearance K für Harnstoff und effektiver Behandlungszeit T der Dialysebehandlung und dem Verteilungsvolumen V des Patienten für Harnstoff definiert ist.

Verfahren und Vorrichtungen zur Messung der Clearance während einer extrakorporalen Blutbehandlung sind aus der DE 39 38 662 A1 (US 5,100,554) und DE 197 47 360 A1 (US 6 156 002) bekannt. Die Bestimmung der Clearance beruht auf der Messung des Elektrolyttransfers bei zwei unterschiedlichen Dialsat-Ionenkonzentrationen. Aus den Druckschriften ist bekannt, dass die Clearance vom Dialysatfluss abhängig ist. Die Clearance ist auch vom Blutfluss abhängig, wobei korrekterweise nur der effektive Blutwasserfluss (Plasmawasser und intrazelluläres Wasser) entscheidend ist.

Die bekannten Dialysegeräte erlauben die manuelle Einstellung unterschiedlicher Dialysierflüssigkeitsraten, beispielsweise 300, 500 und 800 ml/min. Zur Erzielung einer hohen Clearance sind grundsätzlich höhere Dialysatflüsse bei höheren Blutflüssen erforderlich.

Der Dialysator oder Filter einer Blutbehandlungsvorrichtung ist eine austauschbare Einheit, die mit dem Flüssigkeitssystem der Dialysevorrichtung verbunden ist. Das Flüssigkeitssystem der bekannten Blutbehandlungsvorrichtungen umfasst ein Leitungssystem mit einem ersten und einem zweiten Leitungsabschnitt für den Anschluss des Dialysators. Zum Anschluss des Dialysators an das Flüssigkeitssystem werden der erste Leitungsabschnitt mit dem Einlass der Dialysierflüssigkeitskammer und der zweite Leitungsabschnitt mit dem Auslass der Dialysierflüssigkeitskammer des Dialysators verbunden.

Die Hersteller von Dialysatoren und Blutbehandlungsvorrichtungen sehen eine farbliche Kodierung der mit dem Ein- und Auslass zu verbindenden Leitungsabschnitten sowohl auf der Blut- als auch Dialysatseite vor, um dem Anwender den Anschluss im Gegen- oder Gleichstrom zu erleichtern. Diese farbliche Kodierung ist allerdings nicht bei allen Herstellern einheitlich. Daher besteht die Gefahr, dass die Anschlüsse verwechselt werden. Dies wird nachfolgend als nicht ordnungsgemäßer Anschluss bezeichnet. Das Gleiche gilt für eine automatisierte Umkehr der Flussrichtung, beispielsweise durch Umschalten einer im extrakorporalen Blutkreislauf vorgesehen Einrichtung zur Flussumkehr oder durch Umkehr der Förderrichtung der Blutpumpe, da es auch hier zu einem fehlerhaften Anschluss kommen kann.

Wenn der Dialysator nicht im Gegenstrom, sondern Gleichstrom betrieben wird, kann die Effektivität der Blutbehandlung für den Patienten nicht ausreichend sein. Dies ist insbesondere dann problematisch, wenn ein nicht ordnungsgemäßer Anschluss des Dialysators unbemerkt bleiben sollte. Dann besteht die Gefahr, dass der Patient auf Dauer mit nicht ausreichender Effektivität behandelt wird.

Eine Vertauschung der Anschlüsse ist für den Anwender nicht ohne weiteres erkennbar. Eine zu erwartende Reduzierung der Effizienz bei einem Betrieb im Gleichstrom anstelle eines Gegenstrom-Betriebs kann nicht als Indikator für den Gleichstrom-Betrieb herangezogen werden, da eine Verringerung der Dialyseeffizienz auch durch andere Faktoren, beispielsweise Clotting des Dialysators oder Rezirkulation im Gefäßzugang hervorgerufen werden kann und in der Praxis brauchbare Referenzwerte für den Erwartungswert der Dialyseeffizienz fehlen.

Die DE 10 2010 032 980 A1 beschreibt eine Vorrichtung zur Erkennung der Richtung der Flüssigkeitsströmung durch eine Dialysator, die auf der Veränderung einer physikalischen und/oder chemischen Eigenschaft, beispielsweise der Stoffkonzentration, einer in die Blutkammer des Dialysators strömenden Flüssigkeit und der Messung der Veränderung der physikalischen und/oder chemischen Eigenschaft der aus der Blutkammer des Dialysators strömenden Flüssigkeit beruht. Die auf die Veränderung der physikalischen und/oder chemischen Eigenschaft stromauf der Blutkammer des Dialysators zurückzuführende Veränderung der physikalischen und/oder chemischen Eigenschaft der Flüssigkeit stromab der Blutkammer wird vor und nach der Umkehrung der Strömungsrichtung der Flüssigkeit durch die Blutkammer gemessen.

Aus der WO 2012/159734 A1 ist eine Vorrichtung zur Erkennung eines Betriebszustandes einer extrakorporalen Blutbehandlungsvorrichtung bekannt, die über eine Messeinheit zum Messen der Dialyseeffizienz (Clearance) verfügt. Die Messung der Clearance beruht auf der Veränderung der Eigenschaften des frischen Dialysats und der erfolgten Reaktion im verbrauchten Dialysat bei durch den Anwender vorgegebenen Dialysebedingungen, beispielsweise einem vorgegebenen Blutfluss und Dialysatfluss.

Aus der WO 2012/159734 A1 ist auch bekannt, aus dem Massentransferkoeffizienten $K_0A$ als eine Kenngröße für die Effizienz des Dialysators, dem Dialysatfluss $Q_d$ und dem Blutfluss $Q_b$ die Clearance zu berechnen. Dieser die Abhängigkeit der Clearance von der Dialysatrate beschreibende Zusammenhang ist aus der WO 2012/159734 A1 für den Betrieb im Gegenstrom und Gleichstrom bekannt. Allerdings ist der Massentransferkoeffizient in der Praxis nicht bekannt. Eine Übermittlung der Herstellangaben findet in der Regel an die Dialysevorrichtung nicht statt. Selbst wenn der Massentransferkoeffizienten $K_0A$ des Dialysators bekannt sein sollte, so ist doch für die Behandlung der effektive Massentransferkoeffizienten $K_0A$ relevant, in den Blut- und Patientenparameter eingehen.

In der WO 2012/159734 A1 wird als ein fehlerhafter Betriebszustand auch der Betrieb des Dialysators im Gleichstrom anstelle im Gegenstrom beschrieben. Bei einem Blutfluss von 300 ml/min und einem Dialysatfluss von 500 ml/min soll sich bei der Verwendung eines bekannten Dialysators die Clearance beispielsweise um 32% verringern, wenn der Dialysator nicht im Gegenstrom, sondern Gleichstrom betrieben wird. Wenn die Abweichung zwischen gemessener Clearance und für Gegenstrom erwarteter Clearance in dem Bereich liegt, der für Gleichstrom erwartet wird, kann ein Vertauschen der Kupplungen des Dialysators als Ursache der Störung vermutet werden. Allerdings kann allein bei einer Verringerung der Clearance nicht auf einen Betrieb im Gleichstrom geschlossen werden, da eine Verringerung der Clearance auch auf eine Rezirkulation im Gefäßzugang oder andere Ursachen zurückgeführt werden kann.

Die US 2013/0338560A1 beschreibt eine extrakorporale Blutbehandlungsvorrichtung, die über eine Einrichtung zur Veränderung der Strömungsrichtung des durch den Patienten fließenden Blutes verfügt. Zur Veränderung der Strömungsrichtung des Blutes weist die Blutbehandlungsvorrichtung eine Einrichtung auf, die eine Umkehr des Blutflusses in den mittels Kanülen an den Patienten angeschlossenen Leitungsabschnitten des extrakorporalen Blutkreislaufs erlaubt. Während der Blutbehandlung strömt das Blut von der "arteriellen" Kanüle in die Blutkammer des Dialysators und aus der Blutkammer des Dialysators zu der "venösen" Kanüle, während die Dialysierflüssigkeit in entgegengesetzter Richtung durch die Dialysierflüssigkeitskammer des Dialysators strömt. Wenn die Einrichtung zur Veränderung der Strömungsrichtung des durch den Patienten fließenden Blutes umgeschaltet wird, werden zwar der arterielle und venöse Gefäßzugang des Patienten vertauscht, die Strömungsrichtung des Blutes durch die Blutkammer des Dialysators bleibt davon aber unberührt. Die US 2013/0338560A1 befasst sich nicht mit dem Problem, die Strömungsrichtung im Dialysator (Gleichstrom oder Gegenstrom) umzukehren und zu überwachen, sondern festzustellen, ob der arterielle oder venöse Gefäßzugang vertauscht worden sind.

Der Erfindung liegt die Aufgabe zugrunde, eine Vorrichtung zur Erkennung der Richtung der Flüssigkeitsströmung durch einen Dialysator anzugeben, um überprüfen zu können, ob der Dialysator im Gleichstrom oder Gegenstrom betrieben wird.

Darüber hinaus ist eine Aufgabe der Erfindung, eine extrakorporale Blutbehandlungsvorrichtung zu schaffen, mit der die Sicherheit bei der Dialyse erhöht ist.

Die Lösung dieser Aufgaben erfolgt erfindungsgemäß mit den Merkmalen der unabhängigen Patentansprüche. Vorteilhafte Ausführungsformen der Erfindung sind Gegenstand der Unteransprüche.

Ein erster Aspekt der Erfindung liegt darin, zur Erkennung der Richtung der Flüssigkeitsströmung durch den Dialysator die Clearance oder eine für die Clearance charakteristische Größe zu messen und die gemessene Clearance bzw. die gemessene für die Clearance charakteristische Größe mit einem vorgegebenen Grenzwert zu vergleichen, wobei auf die Richtung der Strömung von Blut durch die Blutkammer und Dialysierflüssigkeit durch die Dialysierflüssigkeitskammer im Gegenstrom geschlossen wird, wenn die Clearance oder die für die Clearance charakteristische Größe größer als der vorgegebene Grenzwert ist. Diesem Aspekt liegt die Erkenntnis zugrunde, dass bei einer Blutbehandlung in der Praxis mit einem Betrieb des Dialysators im Gleichstrom Clearance-Werte oberhalb eines bestimmten Grenzwertes nicht mehr erreichbar sind. Daher kann für Clearance-Werte oberhalb eines vorgegebenen Grenzwertes sofort auf einen Betrieb im Gegenstrom geschlossen werden. Folglich kann sofort erkannt werden, ob der in der Praxis vorwiegend gebräuchliche Betrieb des Dialysators im Gegenstrom auch tatsächlich eingestellt ist. Dadurch wird die Sicherheit der Blutbehandlung erhöht.

Der vorgegebene Grenzwert kann für einen bestimmten Bereich, in dem Änderungen des Dialysierflüssigkeitsflusses und/oder Blutflusses in der Praxis zu erwarten sind, ermittelt werden. Dabei können auch die Massentransferkoeffizienten der in der Praxis verwendeten Dialysatoren Berücksichtigung finden.

Für die Messung der Clearance oder einer für die Clearance charakteristischen Größe verfügt die erfindungsgemäße Vorrichtung über eine Messeinheit und für den Vergleich der gemessenen Clearance mit dem vorgegebenen Grenzwert über eine Auswert- und Recheneinheit. Die Messeinheit und die Auswert- und Recheneinheit können selbständige Einheiten bilden oder auch Bestandteil der Messeinrichtung und/oder zentralen Steuereinheit und/oder Rechen- und Auswerteinheit (Mikroprozessor) der Blutbehandlungsvorrichtung sein.

Ein zweiter Aspekt der Erfindung liegt darin, zur Erkennung der Strömungsrichtung die Clearance oder eine für die Clearance charakteristische Größe zu messen und die Flussrate der Dialysierflüssigkeit zu verändern. Hierfür verfügt die erfindungsgemäße Vorrichtung über eine Messeinheit zum Messen der Clearance oder einer für die Clearance charakteristischen Größe vor und nach der Veränderung der Dialysierflüssigkeitsrate und eine Steuereinheit zum Verändern der Flussrate der Dialysierflüssigkeit durch die Dialysierflüssigkeitskammer des Dialysators um einen vorgegebenen Betrag.

Die erfindungsgemäße Vorrichtung nach dem zweiten Aspekt der Erfindung beruht auf dem Vergleich der Veränderung der gemessenen Clearance oder einer für die Clearance charakteristischen Größe mit einem berechneten Erwartungswert der Veränderung der Clearance oder einer für die Clearance charakteristischen Größe für einen Betrieb des Dialysators sowohl im Gegenstrom als auch im Gleichstrom. Auf einen tatsächlichen Betrieb des Dialysators im Gegenstrom wird dann geschlossen, wenn der gemessene Wert für die Veränderung der Clearance bzw. der für die Clearance charakteristischen Größe näher an dem Erwartungswert für die Veränderung der Clearance bzw. der für die Clearance charakteristischen Größe für den Gegenstrom-Betrieb als für den Gleichstrom-Betrieb liegt, während auf einen tatsächlichen Betrieb des Dialysators im Gleichstrom geschlossen wird, wenn der gemessene Wert für die Veränderung der Clearance bzw, der für die Clearance charakteristischen Größe näher an dem Erwartungswert für die Clearance bzw. der für die Clearance charakteristischen Größe für den Gleichstrom-Betrieb als für den Gegenstrom-Betrieb liegt. Der Erfindung nach dem zweiten Aspekt liegt die Erkenntnis zugrunde, dass der Betrag der relativen Änderung der Clearance bei einer Veränderung der Dialysierflüssigkeitsrate bei einem Betrieb im Gleichstrom stets größer ist als im Gegenstrom ist.

Für die Erfindung ist unerheblich, wie der aktuelle Wert der Clearance gemessen wird. Hierzu können sämtliche aus dem Stand der Technik bekannte Verfahren verwendet werden. In diesem Zusammenhang wird unter einer Messung der Clearance auch die Bestimmung der Clearance verstanden, wenn aus gemessenen Größen die Clearance berechnet wird.

Die Rechen- und Auswerteinheit der erfindungsgemäßen Vorrichtung ist derart konfiguriert, dass auf der Grundlage der Veränderung der Clearance oder der für die Clearance charakteristischen Größe, die auf eine Veränderung der Dialysierflüssigkeitsrate um einen vorgegebenen Betrag zurückzuführen ist, auf die Richtung der Strömung von Blut durch die Blutkammer und Dialysierflüssigkeit durch die Dialysierflüssigkeitskammer im Gegenstrom oder Gleichstrom geschlossen wird.

Eine bevorzugte Ausführungsform der Erfindung sieht vor, dass der Betrag der zu erwartenden Veränderung der Clearance, die auf die Veränderung der Dialysierflüssigkeitsrate zurückzuführen ist, oder der für die Clearance charakteristischen Größe auf der Grundlage einer die Abhängigkeit der Clearance oder der für die Clearance charakteristischen Größe von der Dialysierflüssigkeitsrate beschreibenden Zusammenhangs für eine Strömung der Dialysierflüssigkeit im Gegenstrom und Gleichstrom berechnet wird, und dass aus der vor und nach der Veränderung der Dialysierflüssigkeitsrate gemessenen Clearance oder der für die Clearance charakteristischen Größe der Betrag der tatsächlichen Veränderung der Clearance oder der für die Clearance charakteristischen Größe berechnet wird. Auf der Grundlage eines Vergleichs des Betrags der tatsächlichen Veränderung der Clearance oder der für die Clearance charakteristischen Größe mit dem erwarteten Wert der Veränderung der Clearance oder der für die Clearance charakteristischen Größe für den Gegenstrom und den Gleichstrom wird dann auf eine Strömung im Gegenstrom oder Gleichstrom geschlossen. Für den Vergleich des Betrags der tatsächlichen Veränderung und der berechneten Veränderung der Clearance können unterschiedliche Auswertmethoden verwendet werden.

Bei einer bevorzugten Ausführungsform wird der Betrag der Differenz zwischen dem Betrag der tatsächlichen Veränderung der Clearance oder der für die Clearance charakteristischen Größe und dem erwarteten Wert der Veränderung der Clearance oder der für die Clearance charakteristischen Größe für den Gegenstrom und der Betrag der Differenz zwischen dem Betrag der tatsächlichen Veränderung der Clearance oder der für die Clearance charakteristischen Größe und dem erwarteten Wert der Veränderung der Clearance oder der für die Clearance charakteristischen Größe für den Gleichstrom berechnet. Auf eine Strömung im Gleichstrom wird dann geschlossen, wenn der Betrag der Differenz für den Gleichstrom kleiner als der Betrag der Differenz für den Gegenstrom ist, während auf eine Strömung im Gegenstrom geschlossen wird, wenn der Betrag der Differenz für den Gegenstrom kleiner als der Betrag der Differenz für den Gleichstrom ist. Folglich wir geprüft, ob die gemessene Veränderung der Clearance näher an dem Erwartungswert für den Gleichstrom-Betrieb oder Gegenstrom-Betrieb liegt.

Die Messeinheit zum Messen der Clearance oder der für die Clearance charakteristischen Größe weist vorzugsweise Mittel zum Verändern einer physikalischen und/oder chemischen Eigenschaft der in die Dialysierflüssigkeitskammer strömenden Dialysierflüssigkeit und Mittel zum Messen der physikalischen und/oder chemischen Eigenschaft der aus der Dialysierflüssigkeitskammer strömenden Dialysierflüssigkeit auf. Die physikalische und/oder chemische Eigenschaft ist vorzugsweise die Konzentration eines Stoffes in der Dialysierflüssigkeit, wobei die Mittel zum Messen der physikalischen und/oder chemischen Eigenschaft Mittel zum Messen der Stoffkonzentration sind. Für die Erkennung der Durchströmungsrichtung des Dialysators ist grundsätzlich unerheblich, welche physikalische und/oder chemische Eigenschaft verändert wird. Vorzugsweise wird die Na-Konzentration verändert.

Die Veränderung der Stoffmenge kann leicht in der Dialysataufbereitung der extrakorporalen Blutbehandlungsvorrichtung erfolgen. Die Messung der Konzentration eines Stoffes kann mit den bekannten Sensoren erfolgen, die in den bekannten Blutbehandlungsvorrichtungen im Allgemeinen ohnehin vorhanden sind. Daher kann die erfindungsgemäße Vorrichtung leicht in die bekannten Blutbehandlungsvorrichtungen implementiert werden.

Anstelle der Veränderung der Stoffkonzentration kann die Clearance auch mit einer Messeinheit gemessen werden, die eine für die Clearance charakteristische Größe misst, beispielsweise die Absorption von elektromagnetischer Strahlung im Blut, die im UV-Bereich, sichtbaren Bereich oder IR-Bereich liegen kann.

Eine weitere bevorzugte Ausführungsform sieht vor, dass die Rechen- und Auswerteinheit nach der Ermittlung der Strömungsrichtung ein den Betriebszustand im Gegenstrom oder ein den Betriebszustand im Gleichstrom signalisierendes Signal (Daten) erzeugt. Dieses Signal (Daten) kann an eine externe Einheit übermittelt werden. Bei einer besonders bevorzugten Ausführungsform wird das Signal an eine den Betriebszustand anzeigende Anzeigeeinheit übermittelt. Das Signal kann aber auch ein Steuersignal sein, das die zentrale Steuereinheit der Blutbehandlungsvorrichtung empfängt, um einen Eingriff in die Maschinensteuerung vorzunehmen. Der Eingriff in die Maschinensteuerung kann darin liegen, dass die Durchführung der Blutbehandlung verhindert wird. Dadurch ist sichergestellt, dass die Blutbehandlung nur bei ordnungsgemäßem Anschluss des Dialysators möglich ist. Es ist aber auch möglich, als Eingriff in die Maschinensteuerung die Flussrichtung umzukehren, so dass der Dialysator dann ordnungsgemäß betrieben wird. Das Signal kann auch ein Alarmsignal sein, um bei einem fehlerhaften Anschluss des Dialysators einen Alarm zu geben.

Der erste und zweite Aspekt der Erfindung können als unabhängige Kriterien für die Überprüfung der Strömungsrichtung oder in Kombination verwendet werden. Vorzugsweise werden für die Überprüfung der Strömungsrichtung beide Aspekte miteinander kombiniert. Zunächst kann nach dem ersten Aspekt geprüft werden, ob die Clearance oberhalb des vorgegebenen Grenzwertes liegt. Wenn dies der Fall ist, kann eine Überprüfung nach dem zweiten Aspekt entfallen. Die Überprüfung nach dem zweiten Aspekt kann aber der Verifikation der Überprüfung nach dem ersten Aspekt dienen oder auch umgekehrt.

Im Folgenden wird ein Ausführungsbeispiel der Erfindung unter Bezugnahme auf die Zeichnungen näher erläutert.

Es zeigen:

Fig. 1 in stark vereinfachter schematischer Darstellung die wesentlichen Komponenten einer extrakorporalen Blutbehandlungsvorrichtung und

Fig. 2 die relative Änderung der Clearance bei einer Änderung der Dialysierflüssigkeitsrate für den Betrieb des Dialysators im Gleichstrom und im Gegenstrom.

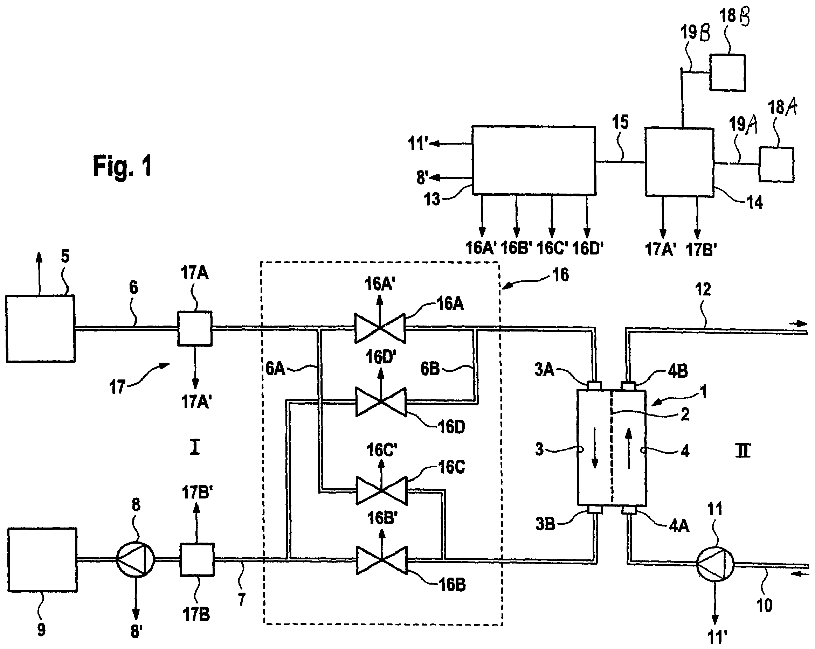

Fig. 1 zeigt in stark vereinfachter schematischer Darstellung nur die für die Erfindung wesentlichen Komponenten einer extrakorporalen Blutbehandlungsvorrichtung. Bei dem vorliegenden Ausführungsbeispiel ist die Vorrichtung zur Erkennung der Richtung der Flüssigkeitsströmung durch den Dialysator der extrakorporalen Blutbehandlungsvorrichtung Bestandteil der Blutbehandlungsvorrichtung. Die Vorrichtung zur Erkennung der Durchströmungsrichtung des Dialysators kann aber auch eine separate Einheit bilden.

Die extrakorporale Blutbehandlungsvorrichtung, die in dem vorliegenden Ausführungsbeispiel eine Hämodialysevorrichtung ist, weist einen Dialysator 1 auf, der durch eine semipermeable Membran 2 in eine Blutkammer 4 und eine Dialysierflüssigkeitskammer 3 getrennt ist. Die Blutkammer 4 weist einen ersten Anschluss 4A und einen zweiten Anschluss 4B auf, während die Dialysierflüssigkeitskammer 3 einen ersten Anschluss 3A und einen zweiten Anschluss 3B aufweist.

Das Flüssigkeitssystem verfügt über eine nur schematisch dargestellte Einrichtung 5, mit der aus Wasser und Konzentraten frische Dialysierflüssigkeit hergestellt wird. Die Einrichtung 5 zur Bereitstellung frischer Dialysierflüssigkeit erlaubt eine kurzfristige Veränderung, insbesondere Erhöhung der Konzentratzusammensetzung, um einen Konzentratbolus zu erzeugen.

Die Einrichtung 5 zur Bereitstellung frischer Dialysierflüssigkeit ist über eine erste Dialysierflüssigkeitsleitung 6 mit dem ersten Anschluss 3A der Dialysierflüssigkeitskammer 3 verbunden. Von dem zweiten Anschluss 3B der Dialysierflüssigkeitskammer 3 führt eine zweite Dialysierflüssigkeitsleitung 7, in die eine Dialysierflüssigkeitspumpe 8 geschaltet ist, zu einem Ablauf 9. Dieser Teil der Blutbehandlungsvorrichtung stellt das Dialysierflüssigkeitssystem I dar.

Von dem Patienten führt eine arterielle Blutleitung 10, in die eine Blutpumpe 11 geschaltet ist, zu dem ersten Anschluss 4A der Blutkammer 4, während von dem zweiten Anschluss 4B der Blutkammer 4 eine venöse Blutleitung 12 abgeht, die zurück zum Patienten führt. Dieser Teil der Blutbehandlungsvorrichtung stellt den extrakorporalen Blutkreislauf II dar.

Während der extrakorporalen Blutbehandlung wird die Dialysierflüssigkeitskammer 3 von Dialysierflüssigkeit und die Blutkammer 4 von Blut durchströmt. Dialysierflüssigkeit und Blut strömen dabei entlang der Membran 2 des Dialysators 1. Um die Effizienz der Behandlung zu steigern, wird der Dialysator 1 im Allgemeinen im Gegenstrom betrieben. Dabei strömen Dialysierflüssigkeit und Blut entlang der Membran in entgegengesetzter Richtung. Der Dialysator kann aber grundsätzlich auch im Gleichstrom betrieben werden.

Die Blutbehandlungsvorrichtung verfügt über eine zentrale Steuereinheit 13, die über Steuerleitungen 8', 11' mit der Dialysierflüssigkeitspumpe 8 und der Blutpumpe 11 verbunden ist.

Die erste und zweite Dialysierflüssigkeitsleitung 6, 7 sind Schlauchleitungen, an denen der Dialysator 1 angeschlossen wird. Zum Anschluss der Schlauchleitungen 6, 7 an den Anschlüssen 3A, 3B des Dialysators 1 dienen nicht dargestellte Anschlussstücke, insbesondere Hansenkupplungen, die im Allgemeinen farblich kodiert sind.

Die Vorrichtung zum Erkennen der Durchströmungsrichtung des Dialysators 1, die bei dem vorliegenden Ausführungsbeispiel Bestandteil der Blutbehandlungsvorrichtung ist, verfügt über eine Rechen- und Auswerteinheit 14, die über eine Datenleitung 15 mit der zentralen Steuereinheit 13 der Blutbehandlungsvorrichtung verbunden ist. Die Rechen- und Auswerteinheit 14 kann aber auch Bestandteil der zentralen Steuereinheit 13 sein. Darüber hinaus verfügt die Vorrichtung zum Erkennen der Strömungsrichtung über eine Steuereinheit zum Verändern der Dialysierflüssigkeitsrate um einen vorgegebenen Betrag, die bei dem Ausführungsbeispiel Bestandteil der zentralen Steuereinheit 13 der Blutbehandlungsvorrichtung ist, aber auch eine separate Einheit sein kann.

Das Dialysierflüssigkeitssystem I kann eine Einrichtung 16 zum Umkehren der Flussrichtung umfassen, die eine Anordnung von Ventilen 16A, 16B, 16C, 16D aufweist. Die Ventile sind vorzugsweise elektromagnetisch oder pneumatisch betätigbare Ventile, die über Steuerleitungen 16A', 16B', 16C', 16D' von der zentralen Steuereinheit 13 der Blutbehandlungsvorrichtung angesteuert werden.

Das Ventil 16A ist in der ersten Dialysierflüssigkeitsleitung 6 und das zweite Ventil 16B in der zweiten Dialysierflüssigkeitsleitung 7 angeordnet. Stromauf des ersten Ventils 16A zweigt von der ersten Dialysierflüssigkeitsleitung 6 ein erster Leitungszweig 6A ab, der zu der zweiten Dialysierflüssigkeitsleitung 7 stromauf des zweiten Ventils 16B führt. In den ersten Leitungszweig 6A ist das dritte Ventil 16C geschaltet. Stromab des ersten Ventils 16A zweigt ein zweiter Leitungszweig 6B von der ersten Dialysierflüssigkeitsleitung 6 ab, der zu der zweiten Dialysierflüssigkeitsleitung 7 stromab des zweiten Ventils 16B führt. In den zweiten Leitungszweig 6B ist das vierte Ventil 16D geschaltet. Dabei beziehen sich die Begriffe stromauf und stromab der Ventile auf die Flussrichtung, wenn die Flüssigkeitsströmung nicht umgekehrt ist.

Im Normal-Betrieb wird der Dialysator 1 im Gegenstrom betrieben. Hierzu öffnet die zentrale Steuereinheit 13 das erste und zweite Ventil 16A, 16B und schließt das dritte und vierte Ventil 16C, 16D. Folglich ist der erste Anschluss 3A der Einlass und der zweite Anschluss 3B der Auslass der Dialysierflüssigkeitskammer 3. Zur Umkehr der Strömungsrichtung schließt die Steuereinheit 13 das erste und zweite Ventil 16A, 16B und öffnet das dritte und vierte Ventil 16C, 16D. Dann ist der erste Anschluss 3A der Auslass und der zweite Anschluss 3B der Einlass der Dialysierflüssigkeitskammer 3.

Die Vorrichtung zum Erkennen der Durchströmungsrichtung des Dialysators verfügt über eine Messeinheit zum Messen der Clearance, die Mittel zum Messen einer physikalischen und/oder chemischen Eigenschaft der Dialysierflüssigkeit aufweist. Bei dem vorliegenden Ausführungsbeispiel ist die physikalische und/oder chemische Eigenschaft der Dialysierflüssigkeit die Konzentration eines Stoffes in der Dialysierflüssigkeit, bspw. die Natrium-Konzentration. Zum Messen der physikalischen und/oder chemischen Eigenschaft sind Mittel 17 vorgesehen, die einen ersten Sensor 17A und einen zweiten Sensor 17B umfassen. Der erste Sensor 17A misst zur Bestimmung der Na-Konzentration die Leitfähigkeit der Dialysierflüssigkeit in der ersten Dialysierflüssigkeitsleitung 6 stromauf des Dialysators 1, während der zweite Sensor 17B die Leitfähigkeit der Dialysierflüssigkeit in der zweiten Dialysierflüssigkeitsleitung 7 stromab des Dialysators 1 misst. Die Sensoren 17A, 17B sind über Datenleitungen 17A', 17B' mit der Rechen- und Auswerteinheit 14 verbunden.

Darüber hinaus sind eine Anzeigeeinheit 18A und eine Alarmeinheit 18B vorgesehen, die über Datenleitungen 19A und 19B mit der Rechen- und Auswerteinheit 14 verbunden sind. Die Anzeigeeinheit 18A zeigt den Betrieb des Dialysators im Gleichstrom oder Gegenstrom an. Die Alarmeinheit 18 gibt einen Alarm, wenn ein nicht ordnungsgemäßer Betrieb des Dialysators 1 festgestellt wird.

Zunächst werden die theoretischen Grundlagen der Erkennung der Durchströmungsrichtung des Dialysators erläutert.

Im Gegenstrom-Betrieb kann aus einer ersten Messung der diffusiven Clearance $K_{diff,1}$ bei Kenntnis von Dialysatfluss $Q_{d,0}$ und Blut(wasser)fluss $Q_{bw}$ der effektive Dialysatorparameter (Massentransferkoeffizient) $K_0A$ berechnet werden (Sargent/Gotch "Principles and Biophysics of Dialysis" in "Replacement of Renal Function by Dialysis"):

$$k_0 A_{\downarrow\uparrow} = \frac{Q_{bw}Q_{d,0}}{Q_{d,0} - Q_b} \ln\left(\frac{\frac{K_{diff,1}}{Q_{d,0}} - 1}{\frac{K_{diff,1}}{Q_{bw}} - 1}\right)$$

Gleichung (1)

Im Gleichstrom-Betrieb lautet der entsprechende Zusammenhang:

$$(k_0 A)_{\uparrow\uparrow} = -\frac{Q_{bw}Q_{d,0}}{Q_{d,0} + Q_{bw}} \ln\left(1 - K_{diff,1}\left(\frac{1}{Q_{bw}} + \frac{1}{Q_{d,0}}\right)\right)$$

Gleichung (2)

Unter der Annahme, dass der Massentransferkoeffizient $K_0A$ bei einer Änderung des Dialysatflusses $Q_d$ konstant bleibt, kann nun bei einer Änderung des Dialysatflusses um $\Delta Q_d$ der Erwartungswert der diffusiven Clearance $\tilde{K}_{diff,2}$ sowohl für den Gegenstrom-Betrieb als auch den Gleichstrom-Betrieb berechnet werden:

$$\left(\tilde{K}_{diff,2}\right)_{\downarrow\uparrow} = Q_{bw}\frac{e^{\gamma_{\downarrow\uparrow}} - 1}{e^{\gamma_{\downarrow\uparrow}} - \frac{Q_{bw}}{Q_{d,0} + \Delta Q_d}}, \gamma_{\downarrow\uparrow} = (k_0 A)_{\downarrow\uparrow}\frac{(Q_{d,0} + \Delta Q_d) - Q_{bw}}{Q_{bw}(Q_{d,0} + \Delta Q_d)}$$

Gleichung (3)

$$\left(\tilde{K}_{diff,2}\right)_{\uparrow\uparrow} = Q_{bw}\frac{1 - e^{-\gamma_{\uparrow\uparrow}}}{1 + \frac{Q_{bw}}{Q_{d,0} + \Delta Q_d}}, \gamma_{\uparrow\uparrow} = (k_0 A)_{\uparrow\uparrow}\frac{(Q_{d,0} + \Delta Q_d) + Q_{bw}}{Q_{bw}(Q_{d,0} + \Delta Q_d)}$$

Gleichung (4)

Die erfindungsgemäße Vorrichtung und das erfindungsgemäße Verfahren können nicht nur für Erkennung der Strömungsrichtung für die Hämodialyse (HD), sondern auch für die Hämodiafiltration (HDF) Verwendung finden. Für den Fall einer HDF-Behandlung mit einer Prädilution oder Postdilution wird aus der durch die Messungen bestimmten Gesamtclearance $K_m$ der diffusive Anteil an der Clearance extrahiert. Dies ist mit der folgenden Gleichung möglich, so dass die Gleichungen (1) bis (4) auch für HDF-Verfahren verwendbar sind.

$$K_{diff} = \frac{Q_{bw} + \kappa Q_s}{Q_b - Q_f - (1-\kappa)Q_s}\left(\frac{Q_{bw} + \kappa Q_s}{Q_b}K_m - Q_f - Q_s\right),$$

$\kappa = 1$ bei HDF-Prädilution

$\kappa = 0$ bei HD und HDF-Postdilution

$Q_{bw}$ bezeichnet den Blutwasserfluss, $Q_b$ den Blutfluss, $Q_d$ den Dialysierflüssigkeitsfluss, $Q_f$ den Filtratfluss und $Q_S$ den Substituatfluss.

Der allgemeine Fall der Hämodiafiltration (HDF) ist in Gross, Maierhofer et al. "Online clearance measurement in high-efficiency hemodiafiltration" (Kidney International (2007) 72, 1550-1553) im Einzelnen beschrieben.

Nach einer zweiten Bestimmung der diffusiven Clearance $K_{diff,2}$ bei einem Dialysatfluss von $Q_{d,0}+\Delta Q_d$ kann nun die reale Veränderung der diffusiven Clearance $\Delta K_{diff}=K_{diff,2}-K_{diff,1}$ für den Gleichstrom-Betrieb und Gegenstrom-Betrieb mit der für den Gleichstrom-Betrieb und Gegenstrom-Betrieb erwarteten Veränderung $\Delta(\tilde{K}_{diff})$ verglichen werden.

$$\Delta\left(\tilde{K}_{diff}\right)_{\downarrow\uparrow} = \left(\tilde{K}_{diff,2}\right)_{\downarrow\uparrow} - \left(\tilde{K}_{diff,1}\right)_{\downarrow\uparrow} bzw.$$

$$\Delta\left(\tilde{K}_{diff}\right)_{\uparrow\uparrow} = \left(\tilde{K}_{diff,2}\right)_{\uparrow\uparrow} - \left(\tilde{K}_{diff,1}\right)_{\uparrow\uparrow}$$

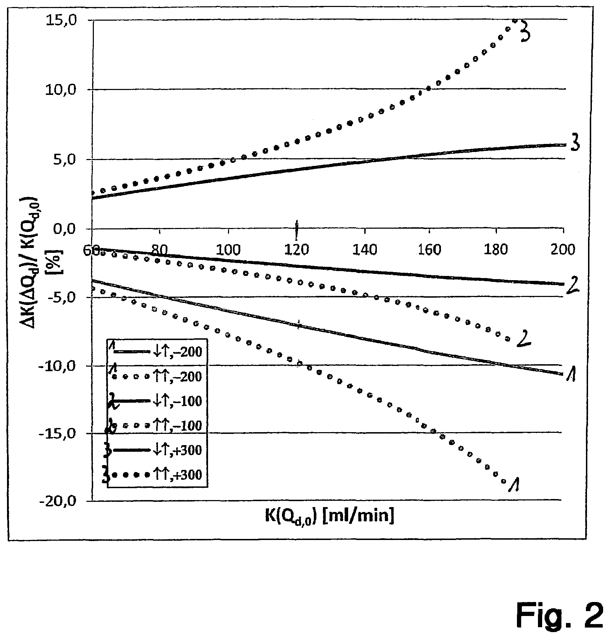

Fig. 2 zeigt die relative Veränderung der Clearance bei einer Veränderung der Dialysierflüssigkeitsrate $Q_d$ um -200 ml/min, -100ml/min (Verringerung) und +300ml/min (Erhöhung) von einer vorgegebenen, ursprünglichen Dialysierflüssigkeitsrate $Q_d$ von 500 ml/min und einer vorgegebenen Blutflussrate $Q_b$ von 300 ml/min bei einem Betrieb des Dialysators 1 im Gegenstrom $\downarrow\uparrow$ und bei einem Betrieb im Gleichstrom $\uparrow\uparrow$ für eine Hämodialysebehanbdlung (HD). Auf der x-Achse ist die Clearance K bei der ursprünglichen Dialysierflüssigkeitsrate von 500 ml/min aufgetragen. Die ermittelten Werte der Clearance K liegen zwischen 60 und 200 ml/min. Es zeigt sich, dass bei einer Blutbehandlung in der Praxis mit einem Betrieb des Dialysators im Gleichstrom Clearance-Werte oberhalb eines Grenzwertes von 185 ml/min nicht mehr erreicht werden. Weiterhin zeigt sich, dass der Betrag der relativen Veränderung der Clearance bei einer Veränderung der Dialysierflüssigkeitsrate bei einem Betrieb im Gleichstrom stets größer als im Gegenstrom ist.

Für typischerweise in der Hämodialyse verwendete Dialysatoren mit einem Massentransferkoeffizienten $K_0A$ von 300-1200 ml/min ist auch im Gleichstrom vor der Veränderung der Dialysierflüssigkeitsrate $Q_b$ eine Clearance K von mindestens 150 ml/min zu erwarten. Damit liegt im vorliegenden Beispiel bei Änderungen der Dialysierflüssigkeitsrate $Q_b$ von -200 bzw. +300 ml/min (auf 300 bzw. 800 ml/min) der Unterschied der Clearanceänderung zwischen Gleich- und Gegenstrom außerhalb der Fehlertoleranz einer leitfähigkeitsbasierten Clearancebestimmung.

Die zentrale Steuereinheit 13 sowie die Rechen- und Auswerteinheit 14 sind derart konfiguriert, dass die einzelnen Verfahrensschritte des erfindungsgemäßen Verfahrens zur Erkennung der Strömungsrichtung durchgeführt werden.

Es wird bei dem Ausführungsbeispiel davon ausgegangen, dass der Dialysator 1 im Gegenstrom betrieben werden soll. Der Gegenstrom-Betrieb ist also der Normal-Betrieb. Dies soll im vorliegenden Ausführungsbeispiel überprüft werden.

Bei einer bestimmten Blutflussrate $Q_b$ und einer bestimmten Dialysierflüssigkeitsrate $Q_{d,0}$, die für die Blutbehandlung vorgegeben werden, misst die Messeinheit die diffusive Clearance $K_{diff,1}$. Hierzu wird durch kurzfristige Veränderung der Konzentratzusammensetzung in der Einrichtung 5 zur Bereitstellung frischer Dialysierflüssigkeit im Dialysierflüssigkeitskreislauf I stromauf des Dialysators ein Konzentratbolus erzeugt, der mit den Sensoren 17A und 17B der Messeinheit stromauf und stromab des Dialysators 1 gemessen wird. Der Sensor 17A stromauf des Dialystors 1 kann auch entfallen, wenn die Größe des Bolus bekannt ist. Die Messeinheit berechnet dann die Clearance $K_{diff,1}$ aus den gewonnenen Messwerten. Die Berechnung der Clearance aus den Messwerten gehört zum Stand der Technik (DE 39 38 662 A1, DE 197 47 360 A1).

Zunächst vergleicht die Rechen- und Auswerteinheit 14 die gemessene Clearance $K_{diff,1}$ mit einem vorgegebene Grenzwert, der oberhalb 160 ml/min, vorzugsweise oberhalb 175 ml/min, besonders bevorzugt oberhalb 185 ml/min liegt. Wenn die Clearance $K_{diff,1}$ oberhalb des Grenzwertes liegt, schließt die Rechen- und Auswerteinheit 14 auf einen Betrieb im Gegenstrom, da ein derart hoher Wert für die Clearance nicht im Gleichstrom-Betrieb zu erreichen ist, was aber nachfolgend nochmals überprüft werden kann. Andernfalls wird auf einen Gleichstrombetrieb geschlossen, was ebenfalls nochmals überprüft werden kann.

Die Dialysierflüssigkeitsrate $Q_d$ wird nunmehr um einen vorgegebenen Betrag verändert, d.h. die Dialysierflüssigkeitsrate $Q_d$ wird erhöht oder verringert, wobei die Blutflussrate $Q_b$ beibehalten wird. Nach der Veränderung der Dialysierflüssigkeitsrate $Q_d$ wird die Clearance $K_{diff,2}$ mit der Messeinheit erneut gemessen.

Die Messwerte $K_{diff,1}$ und $K_{diff,2}$ werden in einem nicht dargestellten Speicher der Rechen- und Auswerteinheit 14 gespeichert. Die Rechen- und Auswerteinheit 14 berechnet aus den Messwerten $K_{diff,1}$ und $K_{diff,2}$ den Betrag der Veränderung der Clearance $\Delta(K_{diff})_{\downarrow\uparrow}$ infolge der Veränderung der Dialysierflüssigkeitsrate von $Q_d$ um den vorgegebenen Betrag $\Delta Q_d$ auf $Q_{d,0} + \Delta Q_d$ unter Beibehaltung der Blutflussrate $Q_b$ für den angenommenen Fall des Betriebs des Dialysators 1 im Gegenstrom:

$$\Delta(K_{diff})_{\downarrow\uparrow} = K_{diff,1} - K_{diff,2}$$

Nach der Bestimmung des Betrags der Veränderung der Clearance $\Delta(K_{diff})_{\downarrow\uparrow}$ auf der Grundlage der Clearancemessungen vor und nach der Veränderung der Dialysierflüssigkeitsrate $Q_d$ wird der Erwartungswert der Clearanceänderung sowohl für den Fall des Gegenstroms $\Delta(\tilde{K}_{diff})_{\downarrow\uparrow}$ als auch für den Fall des Gleichstroms $\Delta(\tilde{K}_{diff})_{\uparrow\uparrow}$ berechnet.

Hierzu wird zunächst nach Gleichung (1) für den Gegenstrom bzw. Gleichung (2) für den Gleichstrom aus der zuvor gemessenen Clearance $K_{diff,1}$, der eingestellten Dialysierflüssigkeitsrate $Q_{d,0}$ und Blutflussrate $Q_b$, sowie dem Blutwasserfluss $Q_{bw}$ der Massentransferkoeffizient $K_0A$ des Dialysators 1 berechnet.

Anschließend wird aus der um den vorgegebenen Betrag $\Delta Q_d$ auf $Q_{d,0} + \Delta Q_d$ erhöhten Dialysierflüssigkeitsrate, der unveränderten Blutflussrate $Q_b$, dem Blutwasserfluss $Q_{bw}$ und dem zuvor bestimmten Massentransferkoeffizienten $K_0A$ nach Gleichung (3) der Erwartungswert der Clearance $(\tilde{K}_{diff,2})_{\downarrow\uparrow}$ für den Gegenstrom nach der Veränderung des Dialysatflusses berechnet, und aus der um den vorgegebenen Betrag $\Delta Q_d$ auf $Q_{d,0} + \Delta Q_d$ erhöhten Dialysierflüssigkeitsrate, der unveränderten Blutflussrate $Q_b$, dem unveränderten Blutwasserfluss $Q_{bw}$ und dem zuvor bestimmten Massentransferkoeffizienten $K_0A$ nach Gleichung (4) der Erwartungswert der Clearance $(\tilde{K}_{diff,2})_{\uparrow\uparrow}$ für den Gleichstrom nach der Veränderung des Dialysatflusses berechnet.

Die Rechen- und Auswerteinheit 14 berechnet daraufhin den Betrag der Differenz zwischen dem Erwartungswert der Clearance $(\tilde{K}_{diff,2})_{\downarrow\uparrow}$ für den Gegenstrom nach der Veränderung der Dialysierflüssigkeitsrate und der vor der Veränderung der Dialysierflüssigkeitsrate gemessenen Clearance, um den Betrag der erwarteten Veränderung der Clearance für den Gegenstrom zu bestimmen, und berechnet den Betrag der Differenz zwischen dem Erwartungswert der Clearance $(\tilde{K}_{diff,2})_{\uparrow\uparrow}$ für den Gleichstrom nach der Veränderung der Dialysierflüssigkeitsrate und der vor der Veränderung der Dialysierflüssigkeitsrate gemessenen Clearance, um den Betrag der erwarteten Veränderung der Clearance für den Gleichstrom zu bestimmen.

Daraufhin berechnet die Rechen- und Auswerteinheit 14 den Betrag der Differenz zwischen der gemessenen Veränderung der Clearance und dem erwarteten Wert der Veränderung der Clearance für den Fall des Gegenstroms und berechnet den Betrag der Differenz zwischen der gemessenen Veränderung der Clearance und dem erwarteten Wert der Veränderung der Clearance für den Fall des Gleichstroms.

Die beiden Differenzwerte werden dann miteinander verglichen. Wenn der Betrag der Differenz für den Gegenstrom kleiner als der Betrag der Differenz für den Gleichstrom ist, schließt die Rechen- und Auswerteinheit 14 auf einen Betrieb im Gegenstrom, was im vorliegenden Ausführungsbeispiel der gewünschte Betrieb ist. Wenn hingegen der Betrag der Differenz für den Gleichstrom kleiner als der Betrag der Differenz für den Gegenstrom ist, wird auf einen Betrieb im Gleichstrom geschlossen, was im vorliegenden Ausführungsbeispiel nicht der gewünschte Betrieb, d.h. ein fehlerhafter Zustand wäre.

Die Rechen- und Auswerteinheit 14 erzeugt darüber hinaus ein den Betriebszustand signalisierendes Steuersignal, das die Anzeigeeinheit 18A empfängt, so dass der Gegenstrom- oder Gleichstrom-Betrieb angezeigt wird.

Wenn der Dialysator 1 nicht ordnungsgemäß an die Dialysierflüssigkeitsleitungen 6, 7 angeschlossen sein sollte, d.h. die Anschlüsse vertauscht sein sollten, erzeugt die Rechen- und Auswerteinheit 14 ein Steuersignal, das die Alarmeinheit 18B empfängt. Die Alarmeinheit 18B gibt dann einen Alarm. Darüber hinaus erzeugt die Rechen- und Auswerteinheit 14 ein Steuersignal, das die zentrale Steuereinheit 13 empfängt. Daraufhin nimmt die Steuereinheit 13 einen Eingriff in die Maschinensteuerung vor. Dieser Eingriff kann darin liegen, dass die Durchführung der Blutbehandlung unterbrochen wird. Alternativ ist es möglich, die Flussrichtung durch Betätigung der entsprechenden Ventile 16A - 16D umzukehren, so dass der Dialysator tatsächlich im Gegenstrom betrieben wird.

## Patentansprüche

**1.** Vorrichtung zur Erkennung der Richtung der Flüssigkeitsströmung durch einen Dialysator (1), der eine von Blut durchströmte Blutkammer (4) und eine von Dialysierflüssigkeit durchströmte Dialysierflüssigkeitskammer (3) aufweist, die von einer semipermeablen Membran (2) voneinander getrennt sind, wobei die Vorrichtung zur Erkennung der Richtung der Flüssigkeitsströmung eine Messeinheit zum Messen der Clearance oder einer für die Clearance charakteristischen Größe aufweist,
**dadurch gekennzeichnet, dass**
eine Rechen- und Auswerteinheit (13) vorgesehen ist, die derart konfiguriert ist, dass die gemessene Clearance oder eine für die Clearance charakteristische Größe mit einem vorgegebenen Grenzwert verglichen wird, wobei auf die Richtung der Strömung von Blut durch die Blutkammer und Dialysierflüssigkeit durch die Dialysierflüssigkeitskammer im Gegenstrom geschlossen wird, wenn die Clearance oder eine für die Clearance charakteristische Größe größer als der vorgegebene Grenzwert ist.

2. Vorrichtung zur Erkennung der Richtung der Flüssigkeitsströmung durch einen Dialysator (1), der eine von Blut durchströmte Blutkammer (4) und eine von Dialysierflüssigkeit durchströmte Dialysierflüssigkeitskammer (3) aufweist, die von einer semipermeablen Membran (2) voneinander getrennt sind, wobei die Vorrichtung zur Erkennung der Richtung der Flüssigkeitsströmung aufweist:

eine Steuereinheit (14) zum Verändern der Flussrate der Dialysierflüssigkeit durch die Dialysierflüssigkeitskammer (3) des Dialysators (1) um einen vorgegebenen Betrag, und
eine Messeinheit (5; 14; 17A, 17B) zum Messen der Clearance oder einer für die Clearance charakteristischen Größe vor und nach der Veränderung der Dialysierflüssigkeitsrate,
**dadurch gekennzeichnet, dass**
eine Rechen- und Auswerteinheit (14) vorgesehen ist, die derart konfiguriert ist, dass auf der Grundlage der Veränderung der Clearance oder einer für die Clearance charakteristischen Größe, die auf eine Veränderung der Dialysierflüssigkeitsrate um einen vorgegebenen Betrag zurückzuführen ist, auf die Richtung der Strömung von Blut durch die Blutkammer (4) und Dialysierflüssigkeit durch die Dialysierflüssigkeitskammer (3) im Gegenstrom oder Gleichstrom geschlossen wird.

3. Vorrichtung nach Anspruch 2, **dadurch gekennzeichnet, dass**
die Rechen- und Auswerteinheit (14) derart konfiguriert ist,
dass der Betrag der zu erwartenden Veränderung der auf die Veränderung der Dialysierflüssigkeitsrate zurückzuführenden Clearance oder der für die Clearance charakteristischen Größe auf der Grundlage eines die Abhängigkeit der Clearance oder der für die Clearance charakteristischen Größe von der Dialysierflüssigkeitsrate beschreibenden Zusammenhangs für eine Strömung der Dialysierflüssigkeit im Gegenstrom und Gleichstrom berechnet wird,
dass aus der vor und nach der Veränderung der Dialysierflüssigkeitsrate gemessenen Clearance oder der für die Clearance charakteristischen Größe der Betrag der tatsächlichen Veränderung der Clearance oder der für die Clearance charakteristischen Größe berechnet wird, und
dass auf der Grundlage eines Vergleichs des Betrags der tatsächlichen Veränderung der Clearance oder der für die Clearance charakteristischen Größe mit dem erwarteten Wert der Veränderung der Clearance oder der für die Clearance charakteristischen Größe für den Gegenstrom und den Gleichstrom auf eine Strömung im Gegenstrom oder Gleichstrom geschlossen wird.

4. Vorrichtung nach Anspruch 3, **dadurch gekennzeichnet, dass**
die Rechen- und Auswerteinheit (14) derart konfiguriert ist, dass
der Betrag der Differenz zwischen dem Betrag der tatsächlichen Veränderung der Clearance oder der für die Clearance charakteristischen Größe und dem erwarteten Wert der Veränderung der Clearance oder der für die Clearance charakteristischen Größe für den Gegenstrom und
der Betrag der Differenz zwischen dem Betrag der tatsächlichen Veränderung der Clearance oder der für die Clearance charakteristischen Größe und dem erwarteten Wert der Veränderung der Clearance oder der für die Clearance charakteristischen Größe für den Gleichstrom berechnet wird, wobei
auf eine Strömung im Gleichstrom geschlossen wird, wenn der Betrag der Differenz für den Gleichstrom kleiner als der Betrag der Differenz für den Gegenstrom ist, und
auf eine Strömung im Gegenstrom geschlossen wird, wenn der Betrag der Differenz für den Gegenstrom kleiner als der Betrag der Differenz für den Gleichstrom ist.

5. Vorrichtung nach einem der Ansprüche 1 bis 4, **dadurch gekennzeichnet, dass** die Messeinheit (5; 14, 17A, 17B) zum Messen der Clearance oder der für die Clearance charakteristischen Größe aufweist:

Mittel (5) zum Verändern einer physikalischen und/oder chemischen Eigenschaft der in die Dialysierflüssigkeitskammer (3) strömenden Dialysierflüssigkeit, und
Mittel (17B) zum Messen der physikalischen und/oder chemischen Eigenschaft der aus der Dialysierflüssigkeitskammer (3) strömenden Dialysierflüssigkeit.

6. Vorrichtung nach Anspruch 5, **dadurch gekennzeichnet, dass** die physikalische und/oder chemische Eigenschaft die Konzentration eines Stoffes in der Dialysierflüssigkeit ist, wobei die Mittel (17A, 17B) zum Messen der physikalischen und/oder chemischen Eigenschaft Mittel zum Messen der Stoffkonzentration sind.

7. Vorrichtung nach einem der Ansprüche 1 bis 6, **dadurch gekennzeichnet, dass** die Rechen- und Auswerteinheit (14) derart konfiguriert ist, dass die Rechen- und Auswerteinheit (14) nach der Ermittlung der Strömungsrichtung ein den Betriebszustand im Gegenstrom oder ein den Betriebszustand im Gleichstrom signalisierendes Signal erzeugt.

8. Vorrichtung nach Anspruch 7, **dadurch gekennzeichnet, dass** eine Anzeigeeinheit (18B) zum Anzeigen des Betriebszustands vorgesehen ist.

9. Extrakorporale Blutbehandlungsvorrichtung mit einem extrakorporalen Blutkreislauf (II), der die Blutkammer (4) eines durch eine semipermeable Membran (2) in die Blutkammer (4) und eine Dialysierflüssigkeitskammer (3) unterteilten Dialysators (1) einschließt, und einem Dialysierflüssigkeitssystem (I), das die Dialysierflüssigkeitskammer (3) einschließt, **dadurch gekennzeichnet, dass** die extrakorporale Blutbehandlungsvorrichtung eine Vorrichtung zur Erkennung der Richtung der Flüssigkeitsströmung nach einem der Ansprüche 1 bis 8 aufweist.

**Claims**

1. Device for detecting the direction of fluid flow through a dialyser (1) that comprises a blood chamber (4), through which blood flows, and a dialysate chamber (3), through which dialysate flows, which are separated from one another by a semi-permeable membrane (2), the device for detecting the direction of fluid flow comprising a measuring unit for measuring the clearance or a value characteristic of the clearance,
   **characterised in that**
   an arithmetic and evaluation unit (13) is provided, which is configured such that the measured clearance or a value characteristic of the clearance is compared to a specified limit value, the direction of blood flow through the blood chamber and dialysate flow through the dialysate chamber being concluded in countercurrent flow if the clearance or a value characteristic of the clearance is greater than the specified limit value.

2. Device for detecting the direction of fluid flow through a dialyser (1) that comprises a blood chamber (4), through which blood flows, and a dialysate chamber (3), through which dialysate flows, which are separated from one another by a semi-permeable membrane (2), the device for detecting the direction of fluid flow comprising:

   a control unit (14) for changing the flow rate of the dialysate through the dialysate chamber (3) of the dialyser (1) by a specified amount, and
   a measuring unit (5; 14; 17A, 17B) for measuring the clearance or a value characteristic of the clearance before and after the change in dialysate rate, **characterised in that**
   an arithmetic and evaluation unit (14) is provided, which is configured such that on the basis of the change in clearance or a value characteristic of the clearance, which value is attributable to a change in dialysate rate by a specified amount, the direction of blood flow through the blood chamber (4) and dialysate flow through the dialysate chamber (3) in co-current flow or in countercurrent flow is concluded.

3. Device according to claim 2, **characterised in that**
   the arithmetic and evaluation unit (14) is configured such that
   the amount of the change to be expected in the clearance or the value characteristic of the clearance attributable to the change in dialysate rate is calculated on the basis of a correlation describing the dependency of the clearance or of the value characteristic of the clearance on the dialysate rate for a dialysate flow in countercurrent flow and co-current flow,
   the amount of the actual change in clearance or in the value characteristic of the clearance is calculated from the clearance or the value characteristic of the clearance measured before and after the change in dialysate rate, and
   a countercurrent flow or co-current flow is concluded on the basis of a comparison of the amount of the actual change in clearance or in the value characteristic of the clearance with the expected value of the change in clearance or in the value characteristic of the clearance for countercurrent flow and co-current flow.

4. Device according to claim 3, **characterised in that**
   the arithmetic and evaluation unit (14) is configured such that the amount of the difference between the amount of the actual change in clearance or in the value characteristic of the clearance and the expected value of the change in clearance or in the value characteristic of the clearance for countercurrent flow and
   the amount of the difference between the amount of the actual change in clearance or in the value characteristic of the clearance and the expected value of the change in clearance or in the value characteristic of the clearance for co-current flow is calculated,

co-current flow being concluded if the amount of the difference for co-current flow is smaller than the amount of the difference for countercurrent flow, and

countercurrent flow being concluded if the amount of the difference for countercurrent flow is smaller than the amount of the difference for co-current flow.

5. Device according to any of claims 1 to 4, **characterised in that** the measuring unit (5; 14, 17A, 17B) for measuring the clearance or the value characteristic of the clearance comprises:

means (5) for changing a physical and/or chemical property of the dialysate flowing into the dialysate chamber (3), and
means (17B) for measuring the physical and/or chemical property of the dialysate flowing out of the dialysate chamber (3).

6. Device according to claim 5, **characterised in that** the physical and/or chemical property is the concentration of a substance in the dialysate, the means (17A, 17B) for measuring the physical and/or chemical property being means for measuring the substance concentration.

7. Device according to any of claims 1 to 6, **characterised in that** the arithmetic and evaluation unit (14) is configured such that, after determining the flow direction, the arithmetic and evaluation unit (14) generates a signal indicating the operating state in countercurrent flow or the operating state in co-current flow.

8. Device according to claim 7, **characterised in that** a display unit (18B) is provided for displaying the operating state.

9. Extracorporeal blood treatment device comprising an extracorporeal blood circuit (II), which includes the blood chamber (4) of a dialyser (1) divided by a semi-permeable membrane (2) into the blood chamber (4) and a dialysate chamber (3), and comprising a dialysate system (I), which includes the dialysate chamber (3), **characterised in that** the extracorporeal blood treatment device has a device for detecting the direction of fluid flow according to any of claims 1 to 8.

**Revendications**

1. Dispositif de détection du sens de l'écoulement de liquide à travers un dialyseur (1), qui présente une chambre de sang (4) traversée par le sang et une chambre de liquide de dialyse (3) traversée par le liquide de dialyse, qui sont séparées l'une de l'autre par une membrane semi-perméable (2), dans lequel le dispositif de détection du sens de l'écoulement de liquide présente une unité de mesure pour la mesure de la clairance ou d'une grandeur caractéristique de la clairance,
**caractérisé en ce que**
une unité de calcul et d'évaluation (13) est prévue, qui est configurée de sorte que la clairance mesurée ou une grandeur caractéristique de la clairance soit comparée avec une valeur limite prédéfinie, dans lequel il est conclu au sens de l'écoulement du sang à travers la chambre de sang et du liquide de dialyse à travers la chambre de liquide de dialyse à contre-courant, lorsque la clairance ou une grandeur caractéristique de la clairance est supérieure à la valeur limite prédéfinie.

2. Dispositif de détection du sens de l'écoulement de liquide à travers un dialyseur (1), qui présente une chambre de sang (4) traversée par le sang et une chambre de liquide de dialyse (3) traversée par le liquide de dialyse, qui sont séparées l'une de l'autre par une membrane semi-perméable (2), dans lequel le dispositif de détection du sens de l'écoulement de liquide présente :

une unité de commande (14) pour la modification du débit du liquide de dialyse à travers la chambre de liquide de dialyse (3) du dialyseur (1) d'une valeur prédéfinie, et
une unité de mesure (5 ; 14 ; 17A, 17B) pour la mesure de la clairance ou d'une grandeur caractéristique de la clairance avant et après la modification du débit de liquide de dialyse,
**caractérisé en ce que**
une unité de calcul et d'évaluation (14) est prévue, qui est configurée de sorte que sur la base de la modification de la clairance ou d'une grandeur caractéristique de la clairance, qui est imputable à une modification du débit de liquide de dialyse d'une valeur prédéfinie, il soit conclu au sens de l'écoulement du sang à travers la chambre de sang (4) et du liquide de dialyse à travers la chambre de liquide de dialyse (3) à contre-courant ou en co-courant.

**3.** Dispositif selon la revendication 2, **caractérisé en ce que**
l'unité de calcul et d'évaluation (14) est configurée de sorte
que la valeur de la modification escomptée de la clairance à imputer à la modification du débit de liquide de dialyse ou de la grandeur caractéristique de la clairance sur la base d'un rapport décrivant la dépendance de la clairance ou de la grandeur caractéristique de la clairance du débit de liquide de dialyse soit calculée pour un écoulement du liquide de dialyse à contre-courant ou en co-courant,
que la valeur de la modification réelle de la clairance ou de la grandeur caractéristique de la clairance soit calculée à partir de la clairance mesurée avant et après la modification du débit de liquide de dialyse ou de la grandeur caractéristique de la clairance, et
qu'il soit conclu à un écoulement à contre-courant ou en co-courant sur la base d'une comparaison de la valeur de la modification réelle de la clairance ou de la grandeur caractéristique de la clairance avec la valeur escomptée de la modification de la clairance ou de la grandeur caractéristique de la clairance pour le contre-courant et le co-courant.

**4.** Dispositif selon la revendication 3, **caractérisé en ce que**
l'unité de calcul et d'évaluation (14) est configurée de sorte que
la valeur de la différence entre la valeur de la modification réelle de la clairance ou de la grandeur caractéristique de la clairance et la valeur escomptée de la modification de la clairance ou de la grandeur caractéristique de la clairance pour le contre-courant et
la valeur de la différence entre la valeur de la modification réelle de la clairance ou de la grandeur caractéristique de la clairance et la valeur escomptée de la modification de la clairance ou de la grandeur caractéristique de la clairance pour le co-courant soient calculées, dans lequel
il est conclu à un écoulement en co-courant, lorsque la valeur de la différence pour le co-courant est inférieure à la valeur de la différence pour le contre-courant, et
il est conclu à un écoulement à contre-courant, lorsque la valeur de la différence pour le contre-courant est inférieure à la valeur de la différence pour le co-courant.

**5.** Dispositif selon l'une quelconque des revendications 1 à 4, **caractérisé en ce que** l'unité de mesure (5 ; 14, 17A, 17B) pour la mesure de la clairance ou de la grandeur caractéristique de la clairance présente :

des moyens (5) de modification d'une propriété physique et/ou chimique du liquide de dialyse s'écoulant dans la chambre de liquide de dialyse (3), et
des moyens (17B) de mesure de la propriété physique et/ou chimique du liquide de dialyse s'écoulant de la chambre de liquide de dialyse (3).

**6.** Dispositif selon la revendication 5, **caractérisé en ce que** la propriété physique et/ou chimique est la concentration d'une substance dans le liquide de dialyse, dans lequel les moyens (17A, 17B) de mesure de la propriété physique et/ou chimique sont des moyens de mesure de la concentration de substance.

**7.** Dispositif selon l'une quelconque des revendications 1 à 6, **caractérisé en ce que** l'unité de calcul et d'évaluation (14) est configurée de sorte que l'unité de calcul et d'évaluation (14) génère après la détermination du sens d'écoulement un signal signalant l'état de fonctionnement en contre-courant ou un signal signalant l'état de fonctionnement en co-courant.

**8.** Dispositif selon la revendication 7, **caractérisé en ce qu'**une unité d'affichage (18B) est prévue pour l'affichage de l'état de fonctionnement.

**9.** Dispositif de traitement sanguin extracorporel avec un circuit sanguin extracorporel (II), qui englobe la chambre de sang (4) d'un dialyseur (1) subdivisé par une membrane semi-perméable (2) en la chambre de sang (4) et une chambre de liquide de dialyse (3), et un système de liquide de dialyse (I), qui englobe la chambre de liquide de dialyse (3), **caractérisé en ce que** le dispositif de traitement sanguin extracorporel présente un dispositif de détection du sens de l'écoulement de liquide selon l'une quelconque des revendications 1 à 8.

Fig. 1

**Fig. 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3938662 A1
- US 5100554 A
- DE 19747360 A1
- US 6156002 A
- DE 102010032980 A1
- WO 2012159734 A1
- US 20130338560 A1

**In der Beschreibung aufgeführte Nicht-Patentliteratur**

- **GROSS ; MAIERHOFER et al.** Online clearance measurement in high-efficiency hemodiafiltration. *Kidney International,* 2007, vol. 72, 1550-1553